Europäisches Patentamt

European Patent Office

Office européen des brevets

(11) Publication number: **0 205 468 B1**

## EUROPEAN PATENT SPECIFICATION

(45) Date of publication of patent specification: **11.03.92**   (51) Int. Cl.⁵: **A61K 7/22**

(21) Application number: **85905829.9**

(22) Date of filing: **28.11.85**

(86) International application number:
**PCT/FI85/00095**

(87) International publication number:
**WO 86/03674 (03.07.86 86/14)**

The file contains technical information submitted
after the application was filed and not included in
this specification

(54) **MOUTH RINSING SOLUTION.**

<table>
<tr><td>

(30) Priority: **18.12.84 FI 845004**

(43) Date of publication of application:
**30.12.86 Bulletin 86/52**

(45) Publication of the grant of the patent:
**11.03.92 Bulletin 92/11**

(84) Designated Contracting States:
**AT BE CH DE FR GB IT LI LU NL SE**

(56) References cited:
**EP-A- 0 024 535**      **EP-A- 0 040 738**
**EP-A- 0 079 611**      **DE-C- 671 334**
**DK-C- 81 674**         **GB-C- 490 384**
**US-A- 3 988 434**      **US-A- 4 357 318**

</td><td>

(73) Proprietor: **LILIUS, Kaj Rainer**
**Viertopolku 5**
**SF-02100 Espoo(FI)**

Proprietor: **TIKKANEN, Matti Haakon August**
**Takojantie 1N**
**SF-02130 Espoo(FI)**

(72) Inventor: **TIKKANEN, Matti Haakon August**
**Takojantie IN**
**SF-022130 Espoo(FI)**

(74) Representative: **Onn, Thorsten et al**
**AB Stockholms Patentbyra Zacco & Bruhn**
**P.O. Box 3129**
**S-103 62 Stockholm(SE)**

</td></tr>
</table>

Rank Xerox (UK) Business Services

## Description

The present invention is concerned with a mouth rinsing solution for therapeutical use in order to prevent the formation of tartar, as well as with the use of the active compound contained in the mouth rinsing solution.

Tartar is a solid, hard deposit formed on the faces of teeth and providing the bacterial flora in saliva with an excellent protected environment, from which some bacteria are capable of causing dental diseases such as caries and different sorts of gingivitis. In the first stage, the formation of tartar requires adherence of so-called plaque onto the tooth faces. Such a plaque is a porous mass consisting of organic materials, in which said mass calcium and phosphate ions diffused from saliva can react into various calcium phosphates, whereby the structure of the plaque is reinforced and hardened by the effect of reactions of crystallization of phosphates into tartar.

Since the above dental diseases are caused by the activity of some detrimental bacteria in the surface parts of teeth and in their recesses, the studies and measures so far have been almost exclusively concentrated on prevention and stopping of the bacterial activity. This has taken place most commonly by means of mouth rinsing, whereat various bacteriocidal agents have been added to the rinsing solution. Even though some additives, such as, e.g., chlorhexidine, have had a positive effect, by their means it has, however, not been possible to solve these problems. During the last decade, by means of fluorine-containing tooth pastes and mouth-washes, and above all by means of fluoridation of drinking water, it has been possible to reduce the occurrence of caries quite efficiently. But even in this way, it has not been possible to prevent the occurrence of the said dental diseases to a sufficient extent. As a new possibility, development of such a vaccine has been suggested as would prevent the generation of the most detrimental one of the bacteria occurring in mouth - Streptococcus mutans. Such experiments are only right at the beginning [1], so that nothing will be known about their results for many years. Moreover, it is to be noticed that the said species of bacteria is not the only one that can produce caries.

The invention to be described in the following is concentrated on prevention of one of the basic factors causing these diseases - formation of tartar. The invention is based both on long-term experiments and on a view of the way in which the formation of tartar begins and develops.

According to this view, a factor preceding the formation of tartar is the formation of so-called plaque on the tooth faces. As was stated above, plaque is formed partly out of organic proteins contained in the liquid in the mouth cavity, partly "by the intermediate of polysaccharides grown by the bacteria contained in the plaque" [ref 1].

Owing to its porous structure, the plaque can absorb both different bacteria and soluble ions contained in the liquid in the mouth cavity, of which said ions the most essential ones are calcium and phosphate ions. Under suitable conditions, the calcium ions and the phosphate ions react with each other and form various calcium phosphates. In the first stage, the formation of such solid phosphate compounds requires the formation of phosphate nuclei, whereinafter the mineralization proper of the plaque, i.e. the formation of macroscopic phosphate crystals, can start.

Thus, mineralization of the plaque, i.e. formation of tartar, can be eliminated if the formation of phosphate nuclei and crystalline phosphate, constituting prerequisites for it, can be prevented.

This idea is also mentioned by the FI Patent No. 50,054, in which it is, however, ascertained that, among other things, solutions that contain EDTA are unsuitable for being used for the purpose concerned.

In DK-C-81 674 products for dental care, e.g. tooth pastes and mouth rinsing solutions, comprising certain Mg containing organic ion exchange complexes, such as Mg-salts of EDTA, are disclosed. These products are said to prevent tartar formation and to reduce the amount of tartar already formed. Sodium salts of EDTA are also discussed therein, but it is maintained that even though these dissolve tartar effectively they give rise to damage on the tooth enamel to such an extent that they cannot be used to prevent formation of and/or to dissolve tartar on teeth.

The present invention is based on long-term experiments and experience concerning the properties and the use of solutions that contain Na-salts of EDTA in the rinsing of tooth faces (in vitro). Thereby, an essential goal has been to develop such a combination of the composition, pH and mode of use of the solution as to prevent formation of tartar efficiently but not to damage the tooth enamel or the other oral faces even during prolonged use.

Contrary to the teachings in the prior art of this field, according to this invention it was unexpectedly found that sodium salts of EDTA can be used in mouth rinsing solutions to prevent tartar formation without giving rise to any simultaneous damage to the teeth.

Accordingly, this invention is related to a mouth rinsing solution therapeutical for use in order to prevent formation of tartar, wherein the solution contains a sodium salt of ethylenediaminetetraacetic acid (EDTA), which complexes calcium ions, so as to prevent formation of phosphate nuclei, **characterized** in that the sodium salt of EDTA is in such a form that the pH of the solution is within the range of 5.5 to 9.0 and in that the quantity of the sodium salt of EDTA in the solution is 1 to 20 %.

On the basis of experience and experiments, it has been found that the compound most suitable for this purpose is such a sodium salt of ethylenediaminetetraacetic acid whose rinsing solution has a pH within the range of 5.5 to 9.0, preferably 6.5 to 8.5. From the practical point of view, 6.7 to 8.0 has proved a suitable pH-range, for two reasons. Firstly, the calcium-binding effect of the EDTA compound is reduced excessively when the pH is lowered below 6.5, which circumstance thereby determines the lower limit of pH. On the other hand, the upper limit of the pH-value must be kept within such a range that the solution does not cause damage in the mouth cavity. It has been noticed in practice that the solution is still fully risk-free when the pH is 8.0. The quantity of ethylenediaminetetraacetic acid in the rinsing solution may be 1 to 20 %, preferably 2 to 15 %. From the practical point of view, 5 to 7 % has proved a suitable quantity.

When the solution is used, it is to be noticed that the rinsings are not started until any earlier tartar has been removed. Thus, the effect is based thereon that the calcium ions diffused into the plaque are bound into complex form before they can react with phosphate ions and form phosphate nuclei and a crystalline phosphate phase in the plaque.

The first experiments were already started in 1979, at which time the inventor started regular rinsings morning and evening after the dentist had removed the tartar as thoroughly as possible. The reason for this initiative was a particularly strong formation of tartar, which had lasted for dozens of years and which has resulted in severe paradentosis. During the years 1979 to 1982, the occurrence of tartar was prevented completely. Towards the end of 1982, the rinsings were discontinued in order to be able to follow new formation of tartar. Formation of tartar was noticed in about three months, whereupon a new series of rinsings was started, upon the removal of this tartar. The result was the same as earlier: there was no more occurrence of tartar.

On the basis of these results, the experiments were continued with clinical tests. In the tests, there were three test groups - totalling 15 test persons.

Test substances:

I: 5 % Na-2-EDTA solution, whose pH was controlled to 7.0. Moreover, the solution contained 250 ppm of NaF.

II: 5 % Na-2-EDTA solution, whose pH was controlled to 7.0.

III: Intensified cleaning method by means of tooth picks and dental floss.

Before the test was started, tartar was removed from every test person by means of an ultrasonic device, and the tooth faces were cleaned by means of a cleaning paste. The groups I and II were given instructions for the use of the test substance, and the group III was taught in treatment at home.

Instructions of use:

The groups I and II have used the test solution as a quantity of about 15 ml, rinsing out in the mouth morning and evening for 1 to 2 minutes, whereupon the mouth was rinsed with water. The group III has cleaned their teeth in an intensified way by mechanical means.

Results:

Group I

Five test persons, of whom four used the test substance regularly; tartar was formed for none of them. With the test person who did not use the test substance regularly, formation of bacterial plaque was noticed, but no calcification of the plaque. Test period 5 weeks.

Group II

Five test persons, all of whom used the test substance regularly. Tartar was formed for none of them; however, two test persons had a thin bacterial plaque.

Test period 4 weeks.

Group III

Five test persons, who had used tooth picks and dental floss, besides brushing, for cleaning the teeth. With three test persons, it was noticed that tartar had been formed on the rear faces of the front teeth of the lower jaw. The remaining two test persons had plaque that was about to be calcified on the rear faces of the front teeth of the lower jaw. Test period 4 weeks.

The test persons in the groups had been chosen by means of a blind test method out of a test material in which, on the basis of earlier experience of treatment, tartar had always been formed within the period of time concerned. Thus, in each group there were different test persons, in respect of the formation of tartar, because saliva studies and other individual differences were not taken into account in this test.

It is well known that the presence of little quantities of fluoride in the mouth has a clear effect on the occurrence of dental caries. This effect is, however, slow, because the fluoride ion must reach an unhindered contact with the enamel face of the teeth. In practice, on the tooth faces, there is either plaque or mineralized plaque, i.e. tartar. The presence of these of course prevents the necessary efficient contact. Accordingly, it is highly advantageous to use little additions of fluoride in the mouth rinsing solution in accordance with the invention, because it is the rinsing solution that can keep the teeth free from tartar. The fluoride additions may be 100 to 300 ppm, but an optimal value is about 200 to 250 ppm.

Since the EDTA agent contained in the mouth rinsing solution must reach a contact as efficient as possible with the calcium ions contained in the plaque, penetration of the solution into the plaque must be aided as efficiently as possible. For this purpose, 0.1 to 0.5 % of sodium salt of lauroyl-sarcosine acid, which is a highly efficient and completely risk-free detergent, is added to the rinsing solution.

The compound in accordance with the invention is not restricted to be used in the way described in the examples only, but a mouth rinsing solution may also consist, e.g., of a concentrate of the said compound diluted with water, or the compound may be added, e.g., to tooth paste.

Reference 1) = TIEDE 2000, No. 6/82, pp. 17 to 22.

**Claims**

1. Mouth rinsing solution for therapeutical use in order to prevent formation of tartar, wherein the solution contains a sodium salt of ethylenediaminetetraacetic acid (EDTA), which complexes calcium ions, so as to prevent formation of phosphate nuclei, **characterized** in that the sodium salt of EDTA is in such a form that the pH of the solution is within the range of 5.5 to 9.0 and in that the quantity of the sodium salt of EDTA in the solution is 1 to 20 %.

2. Mouth rinsing solution as claimed in claim 1, **characterized** in that the sodium salt of EDTA is in such a form that the pH of the solution is within the range of 6.5 to 8.5.

3. Mouth rinsing solution as claimed in claim 2, **characterized** in that the sodium salt of EDTA is in such a form that the pH of the solution is within the range of 6.7 to 8.

4. Mouth rinsing solution as claimed in any of the claims 1 to 3, **characeterized** in that the quantity of the sodium salt of EDTA in the solution is 2 to 15 %.

5. Mouth rinsing solution as claimed in any of the claims 1 to 4, **characterized** in that the quantity of the sodium salt of EDTA in the solution is 5 to 7 %.

6. Mouth rinsing solution as claimed in any of the claims 1 to 5, **characterized** in that 100 to 300 ppm, preferably 200 to 250 ppm, of an inorganic fluoride has been added to the solution.

**Revendications**

1. Solution de rinçage de la bouche pour usage thérapeutique afin de prévenir la formation du tartre, où la solution contient un sel de sodium de l'acide éthylènediaminetétraacétique (EDTA), qui complexe les ions de calcium afin de prévenir la formation des noyaux de phosphate, caractérisée en ce que la sel de sodium de EDTA est sous une forme telle que le pH de la solution soit compris entre 5,5 et 9,0 et en ce que la quantité du sel de sodium de EDTA dans la solution est de 1 à 20%.

2. Solution de rinçage de la bouche selon la revendication 1, caractérisée an ce que le sel de sodium de EDTA est sous une forme telle que le pH de la solution soit compris entre 6,5 et 8,5.

3. Solution de rinçage de la bouche selon la revendication 2, caractérisée en ce que le sel de sodium de EDTA est sous une forme telle que le pH de la solution soit compris entre 6,7 et 8.

4. Solution de rinçage de la bouche selon l'une quelconque des revendications 1 à 3, caractérisée en ce que la quantité du sel de sodium de EDTA dans la solution est de 2 à 15%.

5. Solution de rinçage de la bouche selon l'une quelconque des revendications 1 à 4, caractérisée en ce que la quantité du sel de sodium de EDTA dans la solution est de 5 à 7%.

6. Solution de rinçage de la bouche selon l'une quelconque des revendications 1 à 5, caractérisée en ce que l'on a ajouté, à la solution, 100 à 300 ppm, de préférence 200 à 250 ppm, d'un fluorure inorganique.

**Patentansprüche**

1. Mundspüllösung zur therapeutischen Anwendung, um die Bildung von Zahnstein zu verhindern, worin die Lösung ein Natriumsalz von Ethylendiamintetraessigsäure (EDTA) enthält, das Calciumionen komplexiert, um so die Bildung von Phosphat-Kristallisationskeimen zu verhindern, **dadurch gekennzeichnet**, daß das Natriumsalz von EDTA in einer solchen Form ist, daß der pH der Lösung innerhalb des des Bereichs von 5,5 bis 9,0 ist und daß die Menge des Natriumsalzes von EDTA in der Lösung 1 bis 20% ist.

2. Mundspüllösung nach Anspruch 1, **dadurch gekennzeichnet**, daß das Natriumsalz von EDTA in einer solchen Form ist, daß der pH der Lösung innerhalb des Bereiches von 6,5 bis 8,5 ist.

3. Mundspüllösung nach Anspruch 2, **dadurch gekennzeichnet**, daß das Natriumsalz von EDTA in einer solchen Form ist, daß der pH der Lösung innerhalb des Bereiches von 6,7 bis 8 ist.

4. Mundspüllösung nach einem der Ansprüche 1 bis 3, **dadurch gekennzeichnet**, daß die Menge des Natriumsalzes von EDTA in der Lösung von 2 bis 15% ist.

5. Mundspüllösung nach einem der Ansprüche 1 bis 4, **dadurch gekennzeichnet**, daß die Menge des Natriumsalzes von EDTA in der Lösung von 5 bis 7% ist.

6. Mundspüllösung nach einem der Ansprüche 1 bis 5, **dadurch gekennzeichnet**, daß 100 bis 300 ppm, vorzugsweise 200 bis 250 ppm eines anorganischen Fluorids der Lösung zugesetzt worden sind.